# EUROPÄISCHE PATENTSCHRIFT

(11) **EP 1 073 708 B1**
(45) Veröffentlichungstag und Bekanntmachung des Hinweises auf die Patenterteilung: **14.11.2001**
(21) Anmeldenummer: 99929057.0
(22) Anmeldetag: 27.04.1999
(51) Int. Cl.: C12M 1/16, C12M 1/14, C12M 1/02

(54) **SOLID-STATE-FERMENTER UND VERFAHREN ZUR SOLID-STATE-FERMENTATION**
SOLID-STATE FERMENTER AND METHOD FOR SOLID-STATE FERMENTATION
CUVE ET PROCEDE DE FERMENTATION EN MILIEU SOLIDE

(30) Priorität: 30.04.1998 DE 19820169
(43) Veröffentlichungstag der Anmeldung: 07.02.2001
(73) Patentinhaber: PROPHYTA BIOLOGISCHER PFLANZENSCHUTZ GMBH, 23999 Malchow (DE)
(72) Erfinder: LÜTH, Peter, D-23970 Wismar (DE); EIBEN, Ute, D-23999 Malchow (DE)
(74) Vertreter: Baumbach, Friedrich, Dr.
(86) Internationale Anmeldenummer: DE9901271
(87) Internationale Veröffentlichungsnummer: WO9957239

(56) Entgegenhaltungen:
- FR-A- 1 474 370
- GB-A- 1 156 739
- SU-A- 170 901
- US-A- 3 753 582

## Beschreibung

Die Erfindung betrifft einen Solid-State-Fermenter insbesondere für große Volumina sowie ein Verfahren zur Solid-State-Fermentation.

### Stand der Technik

Die Submers- oder Solid-State-Fermentation dient zur Massenkultur von Mikroorganismen mit dem Ziel, entweder die Mikroorganismen selbst oder Stoffwechselprodukte oder ein mikrobiell umgewandeltes Substrat (z.B. in der Nahrungsmittelindustrie) zu gewinnen. Während heute schon Submersfermenter (Fermenter mit einem flüssigen Nährsubstrat) mit einem Fassungsvermögen bis zu 200.000 Liter gebaut werden, ist es noch nicht gelungen, Solid-State-Fermenter (Fermenter mit einem festen Nährsubstrat) mit wirtschaftlich relevanten Volumina zu konstruieren, die über eine längere Zeit von Kontaminationen durch fremde Mikroorganismen frei gehalten werden können und gleichzeitig eine optimale Kulturführung der Mikroorganismen ermöglichen. Bestimmte filamentöse Pilze benötigen jedoch Oberflächenstrukturen, an denen sie sich entwickeln und sporulieren können. Der mit 50 Litern Fassungsvermögen größte Fermenter zur Produktion filamentöser Pilze bei Verhinderung jeglicher Fremdkontamination steht im INRA in Frankreich (Durand 1997, mündl. Mitteilung). Die Kapazität dieses Fermenters ist jedoch bei weitem nicht ausreichend, um die Produktion von Pilzsporen, die z.B. als biologisches Pflanzenschutzmittel eingesetzt werden können, wirtschaftlich zu gestalten.

Die Solid-State-Fermentation (SSF) ist definiert als Wachstum von Mikroorganismen - gewöhnlich Pilze - auf festen Substraten in einer definierten Gas-Phase, jedoch ohne eine freie Wasser-Phase. Die SSF wurde in bestimmten Gebieten des Orients, Asiens und Afrikas bereits in der Antike zur Herstellung von fermentierter Nahrung, von Enzymprodukten (Koji) oder von Speisepilzen genutzt. In den westlichen Ländern wurden die Anstrengungen seit 1940 auf die Submersfermentation konzentriert; die SSF dagegen wurde lediglich für eine Aufarbeitung von organischen Abfällen verwendet. Aufgrund bestimmter Vorteile gegenüber der Submersfermentation zeigen jedoch neuerlich viele Institute und Unternehmen Interesse an der SSF. Solche Vorteile gegenüber der Submersfermentation sind:
- Möglichkeit einer effektiven Produktion von Sekundärmetaboliten wie Enzymen, Aromastoffen, Duft- und Farbstoffen sowie pharmazeutischen Wirkstoffen
- Möglichkeit der Herstellung von Mikroorganismen als biologische Agenzien in Pflanzenschutzmitteln
- Beseitigung von Toxinen oder anderen schädlichen Substanzen aus Nahrungs- und Futtermitteln bzw. Anreicherung von Proteinen oder Vitaminen in denselben.

Man unterscheidet grundsätzlich 6 Typen von Solid-State-Fermentern:
1. Tray Bioreactor
2. Packed Bed Bioreactor
3. Rotary Drum Bioreactor
4. Swing-Solid-State Bioreaktor
5. Stirred Vessel Bioreactor
6. Air-Solid Fluidized-Bed Bioreactor

Der erste Typ - der Tray Bioreactor -, bei dem das zu fermentierende Substrat in eigens dafür vorgesehene Behälter flach ausgebreitet und in einem speziell dafür klimatisierten Raum ('Koji' -Raum, Ramana Murthy, M.V.; Karanth, N.G.; Raghava Rao, K.S.M.S.: Advance in Applied Microbiology 38 (1993), 99-147) inkubiert wird, ist zwar für die Herstellung großer Produktmengen geeignet, bei diesem Verfahren muß aber eine geringe Kontamination durch Fremdkeime vernachlässigt werden können. Zudem sind Reaktor und Verfahren sehr platz- und arbeitsaufwendig. So muß das zu fermentierende Substrat mittels Handarbeit in den Behältern bewegt werden. Für die Herstellung großer Mengen von Pilzsporen konkurrenzschwacher Arten ist er nicht geeignet.

Bei dem 'Packed Bed Bioreaktor' wird ein feuchtes granulöses Substrat, welches sich in einem geschlossenen Behälter befindet, mit einem Mikroorganismus beimpft, der sich, ohne daß das Substrat bewegt wird, in demselben entwickelt. Dazu muß das Substrat ständig von Luft durchströmt werden. Es treten folgende Probleme auf, die die Verwendung großer Substratmengen von vornherein nicht zulassen.
1. Der Mikroorganismus produziert Wärme (300 kJ pro kg Trockenmasse und Std., Saucedo-Castaneda, G.; Gutierrez-Rojas, M.; Bacquet, G.; Raimbault, M.; Viniegra-Gonzalez, G.: Biotechnologie and Bioengeniering 35 (1990), 802-808), die entweder durch die Außenwand des Behälters oder durch einen erhöhten Luftstrom (Verdunstungskälte) abgeführt werden kann. Dieses ist bei großen Volumina der Behälter nicht möglich. Die Mikroorganismen verlangsamen bei zunehmender Wärmeentwicklung ihr Wachstum und sterben letztlich ab.
2. Durch eine ständige Durchlüftung trocknet das Substrat aus. Der dadurch bedingte 'Schwund' verursacht Luftkanäle, deren Vorhandensein eine gleichmäßige Durchlüftung des Substrates nicht mehr gewährleistet. Das allmähliche Austrocknen des Substrates führt außerdem zu einem verschlechterten Wachstum des Mikroorganismus.

Der 'Rotary Drum Bioreactor' besteht aus einem zylindrischen Behälter, der horizontal angeordnet und drehbar gelagert ist. Der Behälter ist höchstens bis zu einem Drittel seines Volumens mit einem granulösen Kultursubstrat gefüllt, auf dem der Mikroorganismus wächst. Die durch das Wachstum des Mikroorganismus entstehende Wärme kann weitgehend über den z. T. gekühlten Mantel des Behälters abgeführt werden. Dieses geschieht bei der langsamen Drehung des Zylinders, die dazu führt, daß das Substrat immer wieder mit dem Mantel in Berührung kommt und seine Wärme an diesen abgeben kann. Das Verfahren hat aber den Nachteil, daß innerhalb des sich bewegenden Substrates Scherkräfte wirken, die insbesondere zur Zerstörung sich entwickelnder pilzlicher Strukturen (Myzel, Sporenträger, Fruchtkörper) führen. Damit ist das Ziel z. B. einer hohen Sporenausbeute bei vielen Pilzen von vornherein nicht erreichbar. Das Problem der Austrocknung ist bei diesem Fermentertyp weitgehend dadurch gelöst, daß, da die Verdunstung von Wasser aus dem Substrat nicht nötig ist (Verdunstungskälte ist nicht erforderlich), die Belüftung mit feuchter Luft erfolgen kann. Außerdem wäre eine Befeuchtung des Substrates, in dem eine gute Verteilung von freiem Wasser durch die Bewegung gewährleistet ist, auch über Sprühdüsen realisierbar. Bei großen Mengen an Kultursubstrat kommt es bei diesem Fermentertyp allerdings zu weiteren Problemen:
1. Die Konstruktion großer Fermenter ist sehr kostenaufwendig.
2. Durch die kontinuierliche Bewegung des Fermenters kann es zu einer Verklumpung des feuchten Substrates kommen.
3. Es sind Schnittstellen nach außen erforderlich (Luftzu- und Luftabführung, Wasserzuführung), die durch die Rotation des Fermenters leicht zu Quellen einer Fremdkontamination werden können.
Ein ähnlicher Fermenter wie der 'Rotary Drum Bioreactor' ist der 'Swing-Solid-State Bioreactor', nur daß hier die Mischung des Substrates nicht durch eine rotierende Bewegung sondern durch eine schüttelnde Bewegung verursacht wird. Ansonsten gelten die gleichen, bereits genannten Vor- und Nachteile. Eine Volumenbegrenzung dieses Fermentertyps ist jedoch zusätzlich noch dadurch gegeben, daß die Konstruktion des komplizierten Schüttelmechanismus eine Masse des gefüllten Behälters über 100 kg kaum zulassen dürfte.

Den 'Stirred Vessel Bioreaktor' kann man sich als einen geschlossenen Kessel vorstellen, in dem ein Rührwerk läuft. Bei diesem Reaktortyp sind die Probleme bei Verwendung großer Substratmengen vorgezeichnet, da diese nicht mehr gleichmäßig bewegt werden können, ohne daß es zu Zerstörungen in der Substratstruktur kommen würde.

Im 'Air-Solid Fluidized-Bed Bioreactor' wird das Kultursubstrat für die Mikroorganismen ständig in einem Wirbelbett gehalten, wozu ein verhältnismäßig großes Volumen des Reaktorraumes erforderlich ist. Die zur Aufrechterhaltung des Wirbelbettes erforderliche Luft wird im Kreislauf geführt. In der Luft muß ein genau bestimmter Feuchtegehalt aufrechterhalten werden. Das Verfahren erfordert sehr viel Energie zur Aufrechterhaltung des Wirbelbettes. In einem bereits gelaufenen AiF-Projekt (Bahr, d.; Menner, M.: BIOforum 18 (1995), 16 - 21) konnte zwar gezeigt werden, daß die Kultur von Hefezellen in der Wirbelschicht möglich ist.
Dieses wurde jedoch nur in einem relativ kleinen Maßstab und bei im Vergleich zur Submersfermentation relativ kleinen Ausbeuten erreicht. Eine mehrwöchige Kultur filamentöser Pilze an großen Mengen granulösem Kultursubstrat (über 100 kg pro Batch) ist mit dieser Technologie nur mit indiskutabel hohen Kosten möglich.

Weitere Fermenter des Standes der Technik sind zu klein, um mit Ihnen eine wirtschaftlich rentable Menge an Pilzsporen zu gewinnen (EP-A1-0 683 815 und FR 85.08555) oder es ist bei ausreichender Fermenterkapazität nicht möglich, eine Kontamination des Kultursubstrates mit Fremdkeimen über einen längeren Zeitraum auszuschließen (DE 4406632 C1).

Die Aufgabe der vorliegenden Erfindung bestand deshalb darin, einen SSF-Fermenter für große Volumina zu entwickeln und ein Verfahren zur Solid-State-Fermentation bereitzustellen, das eine wirtschaftliche Anwendung der SSF von konkurrenzschwachen Mikroorganismen in großen Fermentern gestattet.

Dabei soll
1. eine Fremdkontamination des Fermenters verhindert werden (Aufrechterhaltung steriler Bedingungen während des gesamten Fermentationsprozesses),
2. die durch den pilzlichen Stoffwechsel entstehende Wärme abgeführt werden, ohne daß es zu einer Austrocknung des Substrates (durch erhöhten Luftdurchsatz und Nutzung der Verdunstungskälte) kommt,
3. das Auftreten von Scherkräften im Fermenter verhindert werden (keine Bewegung des Kultursubstrates) und
4. eine gleichmäßige Belüftung (bei Verhinderung der Austrocknung) und Temperaturführung des Substrates gegeben sein.

In GB-A-1 156 739 wird ein Etagenfermenter beschrieben, der für die aerobe Kultur von Mikroorganismen in festen Substraten geeignet ist. Für die gleichmäßige Verteilung und Durchmischung des Reaktionsmediums weist der Fermenter eine Einrichtung für die seitliche Luft- bzw. Wasserzirkulation auf. Diese Vorrichtung ermöglicht die Aufrechterhaltung steriler Bedingungen, die Abführung entstehender Wärme ohne Austrocknung des Substrates, die Verhinderung des Auftretens von Scherkräften, und die gleichmäßige Belüftung und Temperaturführung des Substrates, ist jedoch für die Beimpfung mit dem zu kultivierenden Mikroorganismus durch Anstauen mit Wasser ungeeignet.

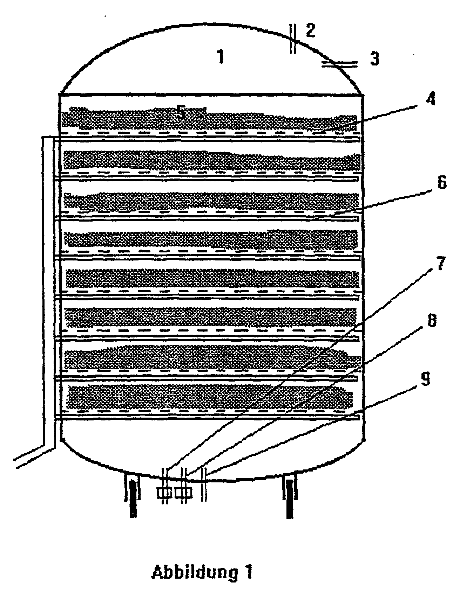

Die Erfindung wird gemäß den Ansprüchen realisiert. Die Aufgabe wurde erfindungsgemäß durch einen Etagenfermenter gelöst, der ein Fassungsvermögen von mindestens 50 Litern, vorzugsweise 500 Litern bis 1000 Litern aufweist, aber auch größere Fassungsvermögen ermöglicht. Die Gesamtkonstruktion besteht aus einem zylindrischen oder ovalen Gefäß (Abbildung 1), das oben durch einen Deckel 1, der gegebenenfalls mit einer Luftableitung 2 sowie einer Öffnung 3 zur Beimpfung des Fermenters versehen ist, verschlossen werden kann.

In dem Gefäß, das als luft- und wasserundurchlässige Hülle konstruiert ist, befinden sich übereinander angeordnete luft- und wasserdurchlässige Etagenböden 4, die zur Aufnahme eines Kultursubstrates 5 für die zu kultivierenden Mikroorganismen dienen.

Das Kultursubstrat besteht entsprechend dem jeweiligen Nährstoffanspruch des zu kultivierenden Mikroorganismus aus unterschiedlichen Materialien. Um eine ausreichende Luftdurchlässigkeit zu gewährleisten, weist dieses Material bevorzugt eine granulöse Struktur auf. Es kann z. B. aus Getreide, Pellets aus Kleie oder sonstigen organischen Abfallprodukten, Abfällen aus der Zuckerproduktion oder mit Nährlösung getränkten Granulaten bestehen.

Die Anzahl der Etagen ist von den Kulturansprüchen des zu kultivierenden Mikroorganismus sowie von der Handhabbarkeit des gesamten Fermenters abhängig. Bei zu vielen Etagen könnte die für das Wachstum der Mikroorganismen erforderliche Sauerstoffversorgung (s.u.) in den oberen Kultursubstratschichten gestört sein. Auch verschlechtern sehr viele Etagen die Handhabbarkeit des Fermenters. Erfindungsgemäß können jedoch 20 oder mehr Etagen im Fermenter untergebracht werden.

Die Etagenböden sind derart mit der Wandung des Gefäßes verbunden, daß weder Luft noch Wasser seitlich an ihnen vorbeiströmen kann. Der Abstand der Etagenböden voneinander ist abhängig von der optimalen Schichtdicke des Kultursubstrates, die wiederum von den Ansprüchen des zu kultivierenden Mikroorganismus bestimmt wird.

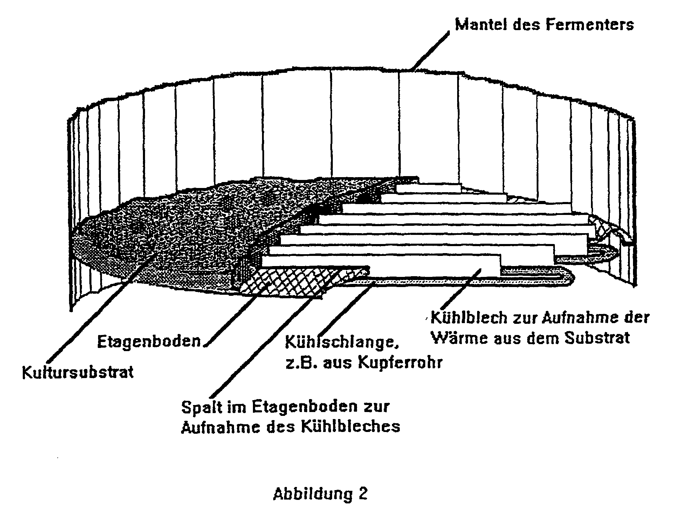

Unter den Etagenböden befinden sich Kühleinrichtungen 6, die als Kühlschlangen oder Kühlplatten konstruiert sein können. Mit ihrer Hilfe wird die Reaktionswärme aus dem Kultursubstrat abgeführt. In einer bevorzugten Variante können von jeder Kühleinrichtung ausgehend, Bleche aus Metall mit einer hohen Wärmeleitfähigkeit durch den jeweiligen Etagenboden hindurch in das Kultursubstrat hineinragen (Abbildung 2). Damit wird die Abführung der Reaktionswärme erleichtert. Zur Entnahme des Kultursubstrates nach Beendigung des Fermentationsprozesses vom Etagenboden wird die Kühleinrichtung mit samt den Kühlblechen vom Etagenboden nach unten abgezogen. Danach kann das mit den Mikroorganismen bewachsene Kultursubstrat entnommen werden, ohne das die Kühlbleche dabei stören.

Die Kühleinrichtungen können auch in einem bestimmten Abstand über den Etagenböden angebracht werden. In diesem Falle sollten sie so installiert werden, daß sie in der Mitte der Kultursubstratschicht verlaufen. Die Anlage der Kühleinrichtungen in den Substratschichten (parallel zu den Etagenböden) ist insbesondere für den Fall vorzusehen, daß im Prozeß der Fermentation sehr viel Reaktionswärme gebildet wird.

Im Boden des Fermenters befindet sich eine Luftzufuhr 7, durch die sterile, angefeuchtete Luft in den Fermenter eingeblasen wird. Die Luft durchströmt alle Substratschichten und verläßt den Fermenter durch den am Deckel angebrachten Luftabfluß 2.

Die sich zwischen den Etagen befindlichen Zwischenräume, in denen auch die Kühleinrichtungen untergebracht sind, gewährleisten eine gleichmäßige Verteilung der Luft im gesamten Fermenter. Steht zur Belüftung des Fermenters keine angefeuchtete Luft zur Verfügung, so kann die Luft auch innerhalb des Fermenters angefeuchtet werden. Dies geschieht, indem wenigstens der unterste Etagenboden nicht mit einem Kultursubstrat sondern mit einem wasseraufnahmefähigen granulösen Material gefüllt ist, das zunächst von der zugeführten Luft durchströmt wird, bevor diese weiter in den Fermenter vordringt. Dadurch wird sie angefeuchtet. Bei einem starken Wasserbedarf des sich entwickelnden Mikroorganismus können mehrere solcher Etagen zur Befeuchtung der Luft in Abständen im. Fermenter installiert sein. Die Menge der einzuführenden Luft ist abhängig vom Sauerstoffbedarf des zu kultivierenden Mikroorganismus. Sie kann zwischen 1 und 100 Liter pro Stunde pro Liter Kultursubstrat variieren.

Zur Beimpfung des Kultursubstrates mit dem zu kultivierenden Mikroorganismus wird der Fermenter, nachdem sein Inhalt zuvor sterilisiert wurde, mit sterilem Wasser bis über die oberste Kultursubstratschicht angestaut. Dazu ist am Fermenterboden ein Wasserzufluß 8, dem ein Sterilfilter vorgeschaltet ist, angebracht. Der Wasserzufluß kann jedoch auch an anderer Stelle des Fermenters (z.B. am Deckel) installiert sein. Nach dem Anstauen wird durch eine dafür vorgesehene Öffnung 3 im Deckel das Inokulum eingebracht. Derartige Öffnungen 3 zur Beimpfung des Fermenters können insbesondere bei sehr vielen Etagen auch zwischen den einzelnen Etagenböden angebracht sein. Im ersten Falle erfolgt die Verteilung des Inokulums im Fermenter ausschließlich beim Wiederablassen des Wassers durch eine dafür vorgesehene Öffnung 9 im Boden des Fermenters.

Das Inokulum (Suspension von Mikroorganismen) durchfließt auf diese Art und Weise alle Kultursubstratschichten und bleibt in ausreichender Menge mit dem anhaftenden Wasser in denselben zurück. Bei zu vielen zu durchströmenden Schichten tritt ggf. abhängig von der Beschaffenheit des Kultursubstrates ein Verdünnungseffekt auf. Das heißt die Mikroorganismen werden durch das zu durchströmende Kultursubstrat abgefiltert, so daß ihre Konzentration im Wasser nach unten hin immer geringer wird. Um dies zu verhindern, können in einer weiteren Variante auch zwischen den Etagenböden Öffnungen zum Einbringen des Inokulums in den Fermenter angebracht sein. Mit ihrer Hilfe kann bereits während des Anstauens des Fermenters mit Wasser Inokulum eingebracht werden, welches dann sowohl mit dem aufwärts als auch mit dem abwärts gerichteten Wasserstrom verteilt wird.

Das für die Beimpfung des Fermenters zu verwendende Inokulum besteht aus einer hochkonzentrierten Suspension kleiner keimungsfähiger Einheiten (vorzugsweise aus Sporen, Konidien oder bakteriellen Keimen) des zu kultivierenden Mikroorganismus.

Unter Voraussetzung einer gleichmäßigen und ausreichenden Beimpfung des Fermentergefäßes ist der Kulturverlauf (Kulturdauer und Produktausbeute) sowie die Qualität des Kulturproduktes (z.B. Pilzsporen) weitgehend von den Parametern der Kulturführung abhängig. Diese besteht in erster Linie in der Zufuhr angefeuchteter Luft und der Temperaturführung. Der Luftvolumenstrom ist mit der Kapazität des Luftsterilfilters abzustimmen. Die Temperaturführung im Fermenter wird mit Hilfe der im Fermenter installierten Kühlung abgesichert. Die Kühlkapazität muß so ausgelegt sein, daß sämtliche Reaktionswärme aus dem Kultursubstrat abgeführt und eine optimale Temperatur für die Kultur des Mikroorganismus aufrecht erhalten werden kann. Die erforderliche Kühlkapazität ist auch von der Schichtdicke und damit vom Volumen des Kultursubstrates abhängig. Je mehr Kultursubstrat für das Wachstum der Mikroorganismen zur Verfügung steht, desto mehr Reaktionswärme wird erzeugt. Daher müssen beide Parameter gemeinsam optimiert werden. Angestrebt wird eine möglichst schnelle Entwicklung der Mikroorganismen sowie eine hohe Produktausbeute, wobei als Produkt je nach Zielstellung der Fermentation, Pilzsporen, Bakterienzellen, Enzyme, Antibiotika, Farbstoffe und weitere Stoffe in Frage kommen.

Es werden zwei Ausführungvarianten des erfindungsgemäßen Fermenters bereitgestellt.

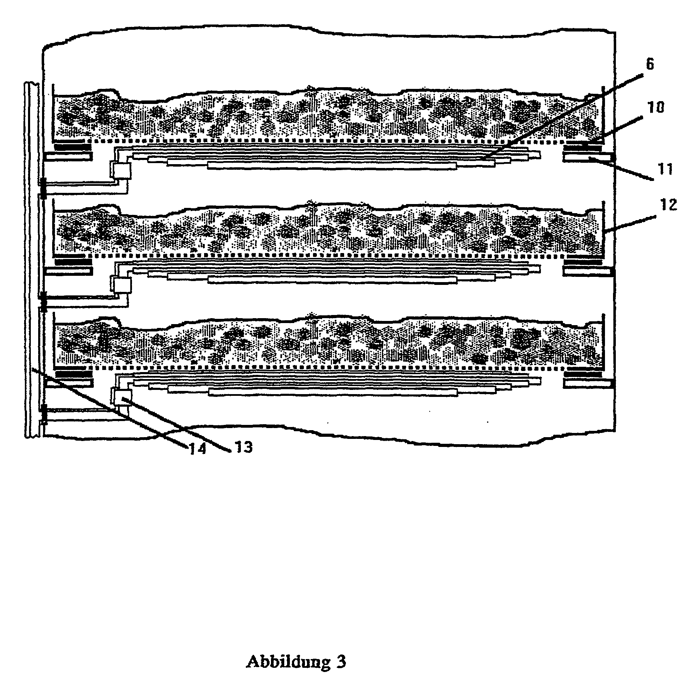

### Variante 1. (Abbildung 3)

Der Fermenter besteht aus einem durchgängigen Zylinder oder einem Prisma, die unten fest verschlossen sind. Der Zylinder (in der Regel ein Kreiszylinder) oder das Prisma können einen Durchmesser von 1 m und mehr haben. Seine Höhe ist durch die technische Handhabbarkeit sowie die Möglichkeit der Aufrechterhaltung optimaler Bedingungen für den zu kultivierenden Mikroorganismus begrenzt. Es sind Höhen von 2 m und mehr realisierbar.

In diesen Zylinder oder das Prisma werden die Etagenböden 4, befüllt mit Kultursubstrat 5, von oben eingebracht. An der Innenseite des Behälters befinden sich Ringe oder, im Falle, daß ein prismatisches Gehäuse verwendet wird, anders geformte Vorrichtungen 11 zur Auflage der Etagenböden. Jeder Ring oder anders geformte Auflagevorrichtung ist mit einer hitzstabilen Dichtung 10, z.B. aus Silikon, versehen, auf die die Etagenböden mit ihrer äußeren Kante aufgelegt werden, wodurch ein luft- und wasserdichter Abschluß zwischen Etagenboden und Gefäßwandung gegeben ist. Die Ringe oder anders geformten Auflagevorrichtungen können aus dem Gehäuse entnommen werden. Die Kühleinrichtung 6 unter dem Etagenboden, die z.B. aus einer Kühlschlange aus Kupferrohr bestehen kann, wird mittels einer Schnellkupplung 13 mit den Leitungen 14 zum Ab- und Zufluß der Kühlflüssigkeit, die sich außerhalb des Fermenters befinden, verbunden. Jeder Etagenboden ist mit einen Rand 12 versehen, dessen Höhe sich nach der Schichtdicke des Kultursubstrates richtet. Dadurch wird verhindert, daß das Kultursubstrat in das Fermentergefäß fällt und diesen verunreinigt.

Der Fermenter wird oben mit dem Deckel 1 fest verschlossen. Er ist als Druckgefäß ausgelegt und kann somit durch die Einleitung von heißem unter Druck stehenden Dampf sterilisiert werden. Es ist daher nicht erforderlich, einen Autoklaven zu nutzen.

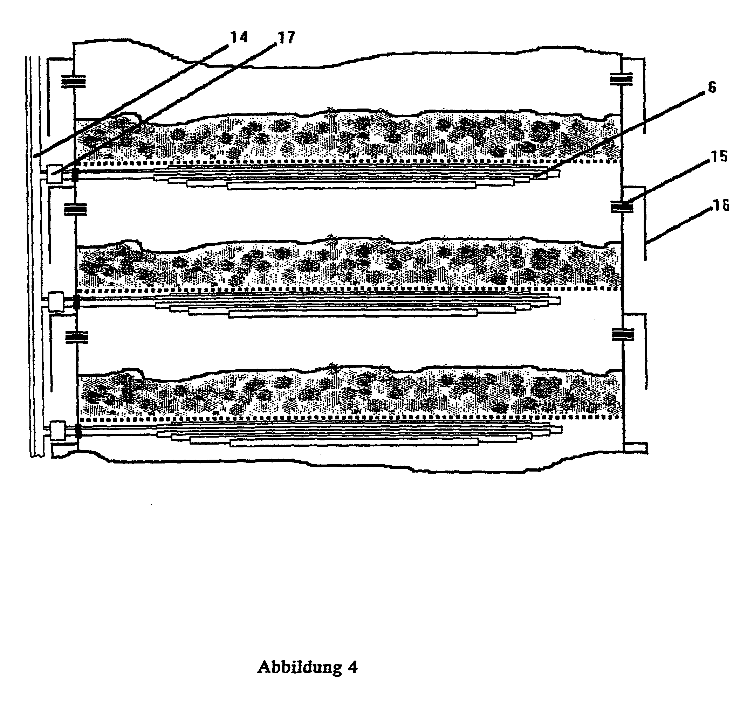

### Variante 2. (Abbildung 4)

Der Fermenter besteht aus mehreren Zylindern oder Prismen mit jeweils einer geringen Höhe (vorzugsweise ca. 7 - 30 cm), die eine kreisförmige, ovale, recht- oder anderseckige Grundfläche haben können. In die einzelnen Zylinder oder Prismen ist jeweils ein luft- und wasserdurchlässiger Boden eingezogen. Unter dem Boden befindet sich die Kühlvorrichtung und auf dem Boden liegt das Substrat zur Kultur der Mikroorganismen. Die Zylinder oder Prismen dienen als Etagen 4 des zusammengesetzten Fermenters. Sie werden übereinander angeordnet und durch hitzestabile Dichtungen 15, die auf den Rändern liegen, gegeneinander abgedichtet. Die erste Etage liegt unten auf dem Fermenterboden auf, und die letzte Etage wird oben von dem Fermenterdeckel verschlossen. Der Fermenter kann so vorzugsweise aus 10 oder mehr Etagen zusammengesetzt werden. Da es schwierig ist, einen solchen zusammengesetzten Fermenter als Druckgefäß zu bauen, erfolgt die Sterilisation des Fermenters und des sich in ihm befindlichen Kultursubstrates in einem Autoklaven. Die Größe des Fermenters ist somit in erster Linie vom Fassungsvermögen des zur Verfügung stehenden Autoklaven abhängig. Er wird daher in den meisten Fällen auf ein Volumen von 500 - 1000 Litern beschränkt bleiben müssen. Während des Autoklavierens ist der Fermenter noch geöffnet, das heißt, die einzelnen Etagen sind leicht (ca. 5 mm) voneinander abgehoben. Dadurch wird eine gute Zufuhr des die Sterilisation bewirkenden heißen Dampfes in den Fermenterinnenraum ermöglicht. Nach dem Autoklavieren wird der Fermenter fest verschlossen. Um eine Kontamination des Fermenters mit Fremdkeimen nach dem Autoklavieren und vor dem Verschließen, also während der Entnahme des Fermenters aus dem Autoklaven, zu verhindern, ist jede Etage mit einem Außenring 16 versehen, der die Aufgabe hat, den zwischen den Etagen im geöffneten Zustand vorhandenen Spalt zu überlappen.

Nach dem Schließen des Fermenters werden die sich unter den Etagenböden befindlichen Kühleinrichtungen 6 mittels einer Kupplung 17 mit den Leitungen 14, welche zum Zu- und Abfluß der Kühlflüssigkeit dienen, verbunden.

In einer bevorzugten Ausführungsvariante besteht ein granulöses Kultursubstrat, an dem sich die Mikroorganismen entwickeln sollen, aus einer 5 - 6 cm dicken Schicht. Es werden bis zu 10 solcher Schichten übereinander angeordnet. Das in Schichten angeordnete granulöse Kultursubstrat lagert jeweils auf einem durchbrochenen und damit luftdurchlässigen Boden, unter dem sich eine Kühlschlange (gewundenes Kupferrohr) befindet, mit deren Hilfe die im Substrat entstehende Wärme abgeführt werden kann. Die Zufuhr von steril gefilterter Luft erfolgt von unten. Durch den hermetischen Abschluß der Etagen zur Seite hin, ist die Luft gezwungen, alle Etagen (Kultursubstratschichten) gleichmäßig zu durchströmen, bevor sie oben den Fermenter wieder verläßt. Auf dem untersten Etagenboden befindet sich eine wassergesättigte Schicht, vorzugsweise SERAMIS-Granulat, durch die die Luft hindurchgeleitet wird, wodurch sie angefeuchtet wird.

Die Sterilisation des Fermenters mit samt des bereits eingebrachten Kultursubstrates erfolgt vorzugsweise durch auf 121 °C erhitzten Dampf, vorzugsweise im Autoklaven, wobei die einzelnen Etagen während des Autoklaviervorganges leicht voneinander abgehoben sind, so daß der heiße Dampf in die Etagen eindringen kann.

| | | |
|---|---|---|
| Weitere Daten | Volumen | 500 Liter |
| | Menge Kultursubstrat | 250 Liter |
| | Luftvolumenstrom | 1500 Liter pro Stunde |
| | Leistung des Kühlsystems | 2,5 kW |

Im Unterschied zu bisher genutzten Typen (Swing-Solid-State-Fermenter oder rotierende Fermenter), wo zur Wärmeabfuhr, Belüftung und Wasserversorgung eine ständige Umschichtung des Kultursubstrates vorgenommen werden muß, ist es bei Anwendung des erfindungsgemäßen Verfahrens nicht mehr erforderlich, das Substrat zu bewegen. Die Lagerung des Kultursubstrates in etagierten Schichten, welche insgesamt von einer geschlossenen Hülle ummantelt sind, bietet folgende Vorteile:
1. Das Eigengewicht des Kultursubstrates führt nicht zur Verdichtung und damit zur Herabsetzung der Luftdurchlässigkeit desselben.
2. Durch die Installation einer Kühlvorrichtung unter den einzelnen Etagen kann entstehende Wärme leicht abgeführt werden.
3. Durch eine relativ geringe Etagenstärke sowie die zwischen den Etagen vorhandenen Räume ist eine gleichmäßige Durchlüftung der Substratschichten gewährleistet.
4. Da die Durchlüftung des Substrates lediglich der Sauerstoffzufuhr sowie der Abfuhr entstehender Gase und nicht der Kühlung des Substrates dient, kann mit einem sehr geringen Luftvolumenstrom gearbeitet werden, der, da die Luft zudem angefeuchtet wird, nicht mehr zum Austrocknen des Substrates führt.
5. Da die Bewegung des Substrates nicht mehr erforderlich ist, kann eine mechanische Zerstörung von Pilzstrukturen (Sporenträger, Fruchtkörper etc.) ausgeschlossen werden.

### Ausführungsbeispiele

- Abbildung 1:: Prinzipzeichnung des Fermenters
- Abbildung 2:: Kühleinrichtung des Fermenters mit Wärmeleitblechen
- Abbildung 3:: Ausschnitt eines Fermenters, bestehend aus einem durchgängigen Zylinder
- Abbildung 4:: Ausschnitt eines zusammengesetzten Fermenters

### Beispiel 1

### Massenkultur von Beauveria brongniartii zum Zwecke der Gewinnung von Pilzkonidien

Der zur Kultur von Beauveria brogniartii verwendete Fermenter hat ein Fassungsvermögen von ca. 50 Litern. Er weist die Form eines Zylinders mit einem Durchmesser von 30 cm und einer Höhe von 70 cm auf. Die Außenhülle des Fermenters besteht aus hitzestabilem Glas. In den Fermenter wurden 8 Etagen eingebaut, deren Böden aus einem Edelstahlsieb mit einer Maschenweite von 3 mm bestehen. Der Abstand der Etagenböden voneinander betrug 8 cm. Der untere Boden wurde mit einer 6 cm starken Schicht SERAMIS-Granulat gefüllt. Die darüber liegenden 7 Etagen enthielten gequetschte Gerstenkörner als Kultursubstrat. Die Schichtdicke des Kultursubstrates betrug ca. 6 cm. Insgesamt wurden 30 Liter Kultursubstrat verwendet.

Der Fermenter wurde mit Hilfe eines Autoklaven sterilisiert. Dazu wurde der Fermenterinhalt mit Hilfe von heißem Dampf für die Dauer einer halben Stunde auf 121 °C erhitzt. Der Deckel des Fermenters war während des Autoklaviervorganges leicht geöffnet, um das Eindringen des Dampfes in den Fermenterinnenraum zu ermöglichen. Er wurde sofort nach dem Autoklavieren geschlossen.

Zur Beimpfung wurde der Fermenter bis über die oberste Kultursubstratschicht mit sterilem Wasser gefüllt. Hierzu wurde ein 500 cm² Capsule des Typs S+S-EXELON PES 20/5 HC (Schleicher und Schuell, Dassel) verwendet. Im Anschluß daran wurde durch eine dafür vorgesehene Öffnung im Deckel das Inokulum eingebracht. Die Beimpfung des Fermenters erfolgte unter einer Laminar-Box. Als Inokulum wurden 100 ml einer Konidiensuspension mit 1 x 10⁹ Konidien pro ml verwendet. Nach der Einbringung des Inokulums über die Oberste Kultursubstratschicht wurde das Wasser durch ein Ventil am Fermenterboden abgelassen. Dadurch wurden alle Substratschichten gleichmäßig mit den Pilzkonidien kontaminiert.

Nach der Beimpfung des Fermenters wurde dieser in einem Raum mit einer Temperatur von 20 °C inkubiert. Es erfolgte ein Anschluß an die Luftzufuhr sowie an das Kühlsystem. Der Luftvolumenstrom betrug während des gesamten Fermentationsvorganges 150 Liter pro Stunde. Als Kühlflüssigkeit wurde Wasser mit einer Vorlauftemperatur von 17 °C verwendet. Die Steuerung der Kühlung war so eingestellt, daß bei einem Überschreiten von 22 °C im Kultursubstrat die Kühlflüssigkeit durch die Kühlschlange gepumpt wurde, bis wieder 20 °C erreicht waren. Auf diese Art und Weise konnte eine durchschnittliche Substrattemperatur von ca. 21 °C während der gesamten Kulturdauer aufrecht erhalten werden.

Ziel der Kultur war eine möglichst hohe Ausbeute an Pilzkonidien. Durch den Glasmantel des Fermenters konnte der Kulturverlauf sehr gut beobachtet werden. Nach etwa 10 Tagen war das gesamte Kultursubstrat von einem weißen Myzel überzogen. Durch die Bildung von Konidien und Konidienträgern veränderte dieses Myzel ab dem 13 Tage sein Erscheinungsbild. Es bekam eine pulverförmige Struktur. Nach etwa 19 Tagen verlor der Fermenter deutlich an Stoffwechselaktivität. Die Wärmeentwicklung verringerte sich, wodurch die Kühlfrequenz deutlich herabgesetzt wurde. Das Kultursubstrat wurde 21 Tage nach der Beimpfung des Fermenters entnommen und die Konidien mittels spezieller Filtrationstechnik aus dem nunmehr vollständig mit Beauveria brogniartii bewachsenen Kultursubstrat gewonnen.
Insgesamt konnten 3,3 x 10¹³ Konidien mit Hilfe des Etagenfermenters gewonnen werden.

### Bezugszeichenliste

- 1: Deckel
- 2: Luftabfluß
- 3: Fermenter
- 4: luftdurchlässiger Etagenboden
- 5: Kultursubstrat
- 6: Kühleinrichtung
- 7: Luftzuführung
- 8: Wasserzuführung
- 9: Wasserabführung
- 10: hitzestabile Dichtung
- 11: Auflagevorrichtung für Etagenboden
- 12: Rand des Etagenbodens
- 13: Schnellkupplung
- 14: Kühlflüssigkeitsab- und -zuflußleitung
- 15: hitzestabile Dichtung
- 16: Außenring
- 17: Kupplung

## Patentansprüche

1. Solid-State-Fermenter
**dadurch gekennzeichnet, daß**
er einen Etagenfermenter darstellt, wobei
mindestens zwei luft- und wasserdurchlässige Etagenböden (4) übereinander angeordnet sind, die so mit der Wandung des Gefäßes verbunden sind, daß weder Luft noch Wasser seitlich vorbeiströmen kann,
sich auf den Etagenböden (4) ein Kultursubstrat (5) für die zu kultivierenden Mikroorganismen befindet,
unter jedem Etagenboden (4) eine Kühleinrichtung (6) angebracht ist und
der Fermenter mit einem Deckel (1) verschlossen ist.

2. Fermenter nach Anspruch 1,
**dadurch gekennzeichnet, daß** er
einen durchgängigen zylindrischen, ovalen, recht- oder anderseckigen Behälter mit mindestens einer Öffnung (3) für das Inokulum darstellt.

3. Fermenter nach Anspruch 1 oder 2,
**dadurch gekennzeichnet, daß**
von der Kühleinrichtung Bleche aus Metall mit einer hohen Wärmeleitfähigkeit durch den jeweiligen Etagenboden hindurch in das Kultursubstrat hineinragen.

4. Fermenter nach einem der Ansprüche 1 bis 3,
**dadurch gekennzeichnet, daß** sich
am Behälter eine Wasserzufuhr (8),
im Boden des Fermenters eine Luftzufuhr (7) sowie ein Abfluß (9) für das Wasser befindet und
am Deckel (1) eine Öffnung zum Luftabfluß (2) vorhanden ist,
die Etagenböden (4), befüllt mit Kultursubstrat (5), nacheinander eingebracht wurden, wozu in der Innenseite des Behälters Ringe oder anders geformte Vorrichtungen (11) zur Auflage der Etagenböden (4) angebracht sind, die mit einer hitzestabilen Dichtung (10) versehen sind und die Etagenböden einen Rand (12) aufweisen, dessen Höhe von der Schichtdicke des Kultursubstrates abhängig ist sowie
die Kühleinrichtungen (6) über eine Schnellkupplung (13) mit Leitungen (14) für Ab- und Zufluß der Kühlflüssigkeit, die sich außerhalb des Fermenters befinden, verbunden sind.

5. Fermenter nach Anspruch 4,
**dadurch gekennzeichnet, daß**
sich der Wasserzufluß (8) im Boden oder Deckel des Fermenters befindet.

6. Fermenter nach einem der Ansprüche 1 bis 5,
**dadurch gekennzeichnet, daß**
sich eine Öffnung für das Inokulum im Deckel befindet und ggf. weitere Öffnungen zur Beimpfung zwischen den einzelnen Etagenböden angeordnet sind.

7. Fermenter nach einem der Ansprüche 1 bis 3,
**dadurch gekennzeichnet, daß**
der Fermenter mehrere zylindrische, ovale oder prismatische Behälter enthält, die als Etagen dienen, und die übereinander angeordnet sind, so daß der erste Behälter unten auf dem Fermenterboden aufliegt und der letzte mit dem Deckel (1) verschlossen ist, und die Behälter durch hitzestabile Dichtungen (15) gegeneinander abgedichtet und mit einem Außenring (16) versehen sind,
jeder dieser Behälter einen luft- und wasserdurchlässigen Boden (4) aufweist, auf dem sich das Kultursubstrat (5) und unter dem sich die Kühlvorrichtung (6) befindet, die mittels einer Kupplung (17) mit Ab- und Zuflußleitungen (14) für die Kühlflüssigkeit, die sich außerhalb des Fermenters befinden, verbunden sind.

8. Fermenter nach einem der Anprüche 1 bis 7,
**dadurch gekennzeichnet, daß**
das Kultursubstrat poröse Granulate, die mit einer Nährlösung versehen werden oder natürliche granulöse Materialien sind.

9. Fermenter nach Anpruch 8,
**dadurch gekennzeichnet, daß**
natürliche granulöse Materialien Getreide, Pellets aus Kleie oder Abfälle aus der Zuckerproduktion sind.

10. Fermenter nach einem der Anprüche 1 bis 9,
**dadurch gekennzeichnet, daß**
sich mindestens auf dem untersten Etagenboden eine Befeuchtungsschicht befindet.

11. Fermenter nach Anpruch 10,
**dadurch gekennzeichnet, daß**
die Befeuchtungsschicht ein wasseraufnahmefähiges, granulöses Material mit einem extrem hohen Porenvolumen ist.

12. Verfahren zur Solid-State-Fermentation mit einer Vorrichtung gemäß einem der Ansprüche 1 bis 11,
**dadurch gekennzeichnet, daß**
ein Kultursubstrat, das sich auf mehreren Etagenböden im Fermenter befindet, gleichmäßig beimpft, vollständig von einem relativ geringen Luftvolumenstrom durchströmt und mittels Kühlsystem die optimale Temperatur für den jeweiligen Kulturvorgang eingestellt wird.

13. Verfahren nach Anspruch 12,
**dadurch gekennzeichnet, daß**
die Beimpfung mit dem zu vermehrenden Mikroorganismus durch Anstauen des Fermenters mit Wasser, dem die Keime in ausreichender Menge beigegeben werden, in einer Weise erfolgt, daß alle Schichten sowohl beim Anstauen als auch beim Abfließen gleichmäßig durchströmt und beimpft werden.

14. Verfahren nach einem der Ansprüche 12 bis 13,
**dadurch gekennzeichnet, daß**
die Kühlkapazität in Abhängigkeit des Volumens des Kultursubstrates so bemessen wird, daß sämtliche Reaktionswärme aus dem Kultursubstrat abgeführt wird.

## Claims

1. Solid state fermenter
**characterized by**
the representation of a module fermenter, which has at least two module bases (4) that are permeable for air and water and that are arranged on top of each other, which are connected to the wall of the vessel, that neither air nor water can pass laterally,
the location of a cultivation substrate (5) on the module bases (4) for the micro-organisms, which are to be cultivated,
a cooling unit (6) mounted below every module base (4) and
a fermenter closed with a lid (1).

2. fermenter according to claim 1,
**characterized by**
representing a patent cylindrical, oval, rectangular or differently angular container with at least one orifice (3) for the inoculum.

3. fermenter according to claim 1 or 2,
**characterized by**
metal plates with a high thermal conductivity that protrude from the cooling device, through the respective module base into the cultivation substrate.

4. fermenter according to one of the claims 1 to 3,
**characterized by**
the existence of a water supply (8) on the container,
of an air-inlet (7) on the bottom of the fermenter as well as of a water discharge (9) and
the existence of an orifice on the lid (1) for the outlet of air (2),
module bases (4) filled with cultivation substrate (5), that were placed one after the other, for which rings or devices of different shape (11) are mounted in the interior of the container for the support of the module bases (4), that are equipped with a heat resistant seal (10) and module bases having an edge (12), whose height depends of the thickness of the layer of the cultivation substrate as well as
cooling devices (6) that are connected via a quick coupling (13) with pipes (14) for discharge and supply of the cooling liquid, which are outside of the fermenter.

5. fermenter according to claim 4,
**characterized by**
the mounting place of the water supply (8) in the bottom or in the lid of the fermenter.

6. according to one of the claims 1 to 5,
**characterized by**
an orifice for the inoculum in the lid and if necessary further orifices for the inoculation arranged between the single module bases.

7. fermenter according to one of the claims 1 to 3,
**characterized by**
the fermenter containing several cylindrical, oval or prismatic containers, that serve as modules, and that are arranged on top of each other in such a way that the first container is supported below on the fermenter bottom and that the last one is closed with a lid (1), and that the containers are sealed from each other with heat resistant seals (15) and are equipped with an exterior ring (16),
the existence of a base (4) that is permeable for air and water in each of the containers, where the cultivation substrate (5) is located and below which cooling device (6) is, which is connected by means of a coupling (17) with the inflow and
outflow pipes (14) for the cooling liquid, that are outside of the fermenter.

8. fermenter according to one of the claims 1 to 7,
**characterized by**
the cultivation substrate being porous granulates with an added nutrient solution or being natural granular materials.

9. fermenter according to claim 8,
**characterized by**
cereals, pellets of bran or waste from the sugar production are natural granular materials.

10. fermenter according to one of the claims 1 to 9,
**characterized by**
the existence of a moistening layer, which is at least on the lowest module base.

11. fermenter according to claim 10,
**characterized by**
the moistening layer being a granular material capable of absorbing water with an extremely high pore volume.

12. procedure for solid state fermentation with a device according to one of the claims 1 to 11,
**characterized by**
a cultivation substrate, which is in several module bases in the fermenter, evenly inoculated, completely circulated by a relatively low air volume flow and for which the optimum temperature is adjusted by means of the cooling system for the respective cultivation procedure.

13. procedure according to claim 12,
**characterized by**
a realization of the inoculation with the micro-organism, which is to be multiplied by filling up water in the fermenters, to which the germs are added in a sufficient amount and in a manner, that all the layers are evenly circulated and inoculated during the filling as well as during the discharging.

14. procedure according to one of the claims 12 to 13,
**characterized by**
a dimensioning of the cooling capacity in dependence of the volume of the cultivation substrate in such a manner that the whole heat of reaction is evacuated from cultivation substrate.

## Revendications

1. Un fermentateur solid-state
se caractérisant par le fait qu'
il représente un fermentateur à étages, deux fonds d'étages perméables à l'air et à l'eau (4) au moins étant placés les uns au-dessus des autres, étant en contact avec la paroi du récipient de sorte que ni air ni eau ne puissent circuler sur les côtés,
un substrat de culture (5) pour les microorganismes à cultiver se trouvant sur les fonds d'étages (4),
un dispositif de refroidissement (6) étant installé sous chaque fond d'étage (4) et le fermentateur étant fermé à l'aide d'un couvercle (1).

2. Un fermentateur selon la revendication 1,
se caractérisant par le fait qu'
il présente un récipient cylindrique, ovale, rectangulaire ou autrement angulaire en continu avec au moins une ouverture (3) pour l'inoculum.

3. Un fermentateur selon la revendication 1 ou 2,
se caractérisant par le fait que
à partir du dispositif de refroidissement, des tôles en métal possédant une conductibilité thermique élevée pénètrent au travers des fonds d'étages respectifs jusque dans le substrat de culture.

4. Un fermentateur selon l'une des revendications 1 à 3,
se caractérisant par le fait qu'
une amenée d'eau (8) se trouve sur le récipient,
une amenée d'air (7) ainsi qu'une évacuation (9) pour l'eau
se trouve dans le fond du fermentateur et
qu'il existe dans le couvercle (1) une ouverture pour l'évacuation de l'air (2),
que les fonds d'étages (4), remplis de substrat de culture (5), ont été intégrés les uns après les autres, pour cette réalisation ont été placés sur la face intérieure du récipient en guise de support pour les fonds d'étage (4), des bagues ou des dispositifs d'autres formes (11) munis d'un joint résistant à la chaleur (10) et que les fonds d'étage disposent d'une bordure (12) dont la hauteur dépendra de l'épaisseur de couche du substrat de culture ainsi que
les dispositifs de refroidissement (6) sont reliés par un raccord rapide (13) aux conduites (14) d'évacuation et d'amenée du liquide refroidissant, conduites qui se trouvent à l'extérieur du fermentateur.

5. Un fermentateur selon la revendication 4,
se caractérisant par le fait que
l'amenée d'eau (8) se trouve dans le fond ou le couvercle du fermentateur.

6. Un fermentateur selon l'une des revendications 1 à 5,
se caractérisant par le fait qu'
une ouverture pour l'inoculum se trouve dans le couvercle et le cas échéant d'autres ouvertures pour l'inoculation sont placées entre les différents fonds d'étage.

7. Un fermentateur selon l'une des revendications 1 à 3,
se caractérisant par le fait que
le fermentateur contient plusieurs récipients cylindriques, ovales ou prismatiques qui jouent le rôle d'étages, et qui sont placés les uns au-dessus des autres de sorte que le premier récipient repose en bas sur le fond du fermentateur et que le dernier soit fermé par le couvercle (1), et que les récipients sont étanchéisés l'un contre l'autre par des joints résistants à la chaleur (15) et sont munis d'une bague extérieure (16), que chacun de ces récipients présente un fond perméable à l'air et à l'eau (4), fond sur lequel se trouve le substrat de culture (5) et sous lequel se trouve le dispositif de refroidissement (6) qui sont reliés au moyen d'un manchon (17) avec les conduites d'évacuation et d'amenée (14) du liquide refroidissant, qui se trouvent à l'extérieur du fermentateur.

8. Un fermentateur selon l'une des revendications 1 à 7,
se caractérisant par le fait que
le substrat de culture se compose de granules poreuses qui seront garnies d'un bouillon de culture, ou de matériaux granuleux naturels.

9. Un fermentateur selon la revendication 8,
se caractérisant par le fait que
les matériaux granuleux naturels sont des céréales, des pellets de son ou des déchets issus de la production du sucre.

10. Un fermentateur selon l'une des revendications 1 à 9,
se caractérisant par le fait que
au moins sur le fond d'étage le plus bas se trouve une couche d'humidification.

11. Un fermentateur selon la revendication 10,
se caractérisant par le fait que
la couche d'humidification se compose d'un matériel granuleux aux propriétés hygroscopiques avec un volume poreux extrêmement élevé.

12. Un procédé de fermentation solid-state avec un dispositif conforme à l'une des revendications 1 à 11,
se caractérisant par le fait qu'
un substrat de culture qui se trouve sur plusieurs fonds d'étage dans un fermentateur, sera inoculé de façon homogène, traversé intégralement par un volume d'air relativement faible et que la température optimale pour le processus de culture correspondant sera réglée au moyen d'un système de refroidissement.

13. Un procédé selon la revendication 12,
se caractérisant par le fait que
l'inoculation avec le microorganisme à multiplier se fait par accumulation d'eau dans le fermentateur, eau à laquelle les germes seront joints en quantité suffisante, de sorte que toutes les couches soient traversées et inoculées de façon homogène aussi bien lors de l'accumulation d'eau que lors de l'évacuation d'eau.

14. Un procédé selon l'une des revendications 12 à 13,
se caractérisant par le fait que
la capacité de refroidissement est calculée en rapport au volume du substrat de culture de sorte que toute la chaleur de réaction en provenance du substrat de culture soit évacuée.
